# EUROPEAN PATENT APPLICATION

(11) **EP 4 691 447 A1**
(43) Date of publication of application: **11.02.2026**
(21) Application number: 25194959.0
(22) Date of filing: 08.08.2025
(51) Int. Cl.: A61J 15/00

(54) **CLEANING DEVICE FOR A FEEDING TUBE CONNECTOR**

(30) Priority: 09.08.2024 US 202463681305 P
(71) Applicant: Eberley, Mathew, Greely, Ontario K4P 1G3 (CA)
(72) Inventor: Eberley, Mathew, Greely, Ontario K4P 1G3 (CA)
(74) Representative: Morabito, Sara

(57) **Abstract**

A cleaning device for cleaning a feeding tube connector comprising a suction connector and a cleaning member. The cleaning member is adapted to clean debris from the feeding tube connector. The suction connector, when connected to a suction tube, allows a user to suck debris from the feeding tube connector.

## Description

### FIELD

The invention relates to the field of cleaning devices, and more specifically to improved cleaning devices for cleaning feeding tube connectors.

### BACKGROUND

Feeding tubes deliver nutrition to neonates and other healthcare patients that cannot, for a variety of medical reasons, eat or drink by mouth. Feeding tube connectors, such as those having ISO Standard 80369-3, can ensure a compatible connection between feeding tubes and a variety of medical devices. Unfortunately, the internal geometry of feeding tube connectors often leads to an accumulation of organic debris therein, such as food particles, breastmilk, formula, and medication.

Existing devices that clean feeding tube connectors have several drawbacks. When a previously disclosed cleaning device is connected to a feeding tube connector, the opening of the feeding tube connector is plugged by a male portion of the cleaning device, thereby introducing unwanted particles into the feeding tube connector. Additionally, previously disclosed cleaning devices are limited to structures that brush or scrape away organic debris. On their own, these structures are sometimes insufficient to clear all organic debris. Furthermore, the use of brushes to clean the opening of feeding tube connectors is problematic. Brushes pick up and retain contaminants that can then be transferred to feeding tube connectors during cleaning. Additionally, brush bristles are likely to get caught at the opening of feed tube connectors and will make it more difficult to engage the cleaning device with the connector. As such, there is a need for a brushless cleaning device that does not plug feeding tube connectors during operation, and that provides an additional cleaning mechanism on top of brushing or scraping away organic debris.

### SUMMARY

In an aspect, the present disclosure provides a cleaning device for use with a feeding tube connector, the cleaning device comprising: a suction connector positioned at a first end of the cleaning device, the suction connector having a first opening at a distal end of the suction connector, the suction connector being adapted for insertion into a suction tube; a cleaning member positioned at a second end of the cleaning device, the second end being opposite to the first end, the cleaning member having a second opening at a distal end of the cleaning member, the cleaning member being adapted to clean the feeding tube connector; and, a first passage extending from the first opening of the suction connector to the second opening of the cleaning member, the first passage allowing debris to be sucked from the feeding tube connector.

In another aspect, the present disclosure provides a cleaning device further comprising a barrier positioned between the suction connector and the cleaning member, the barrier being shaped to stop advancement of the suction tube.

In another aspect, the present disclosure provides a cleaning device wherein a first diameter of the barrier is wider than a second diameter of an opening of the suction tube.

In another aspect, the present disclosure provides a cleaning device further comprising at least one protrusion adapted to scrape the debris from a moat of the feeding tube connector.

In another aspect, the present disclosure provides a cleaning device further comprising a funnel extending from the barrier, the funnel having: a dish portion; a third opening within the dish portion, the third opening being adapted to be occluded by a user; a neck connecting the dish portion to the barrier; and, a second passage extending from the third opening to the first passage.

In another aspect, the present disclosure provides a cleaning device, wherein the dish portion curves inwardly toward the third opening, wherein the second passage is in fluid communication with the third opening, wherein the first passage is in fluid communication with the second passage, and wherein suction power within the first passage is increased when the third opening is occluded.

In another aspect, the present disclosure provides a cleaning device, wherein the suction connector comprises at least one convex segment, the at least one convex segment being shaped to create a sealed connection with the suction connector.

In another aspect, the present disclosure provides a cleaning device, wherein the suction connector comprises at least two convex segments, and wherein diameters of the at least two convex segments decrease as they are positioned further from the barrier.

In another aspect, the present disclosure provides a cleaning device, wherein a diameter of each of the at least one convex segment tapers towards the distal end of the suction connector.

In another aspect, the present disclosure provides a cleaning device further comprising a shield, the shield comprising: a wall portion positioned within the first passage, the wall portion preventing debris in the first passage from falling into the feeding tube connector; at least two bridge members connecting the wall portion to an inner surface of the first passage; and, at least two gaps formed between the at least two bridge members, the at least two gaps allowing debris to be sucked through the first passage.

In another aspect, the present disclosure provides a cleaning device, wherein each of the at least two gaps are positioned between the wall portion and the inner surface of the first passage to allow debris to travel along the first passage from the cleaning member to the suction connector.

In another aspect, the present disclosure provides a cleaning device, wherein the cleaning member further comprises a cylindrical portion, and wherein a distal end of the at least one protrusion extends beyond a distal end of the cylindrical portion.

In another aspect, the present disclosure provides a cleaning device, wherein the at least one protrusion is positioned on the cylindrical portion such that rotation of the cleaning member allows the at least one protrusion to scrape off the debris from the moat.

In another aspect, the present disclosure provides a cleaning device, wherein the at least one protrusion comprises at least one distal edge, and wherein the at least one distal edge is smoothed to prevent injury to a patient.

### BRIEF DESCRIPTION OF THE DRAWINGS

The following figures serve to illustrate various embodiments of features of the disclosure. These figures are illustrative and are not intended to be limiting.
Figure 1 is a perspective view of a cleaning device according to an embodiment of the present disclosure;
Figure 2 is another perspective view of a cleaning device according to an embodiment of the present disclosure;
Figure 3 is another perspective view of a cleaning device according to an embodiment of the present disclosure, the cleaning device being positioned between a suction tube and a feeding tube connector;
Figure 4 is a side view of a cleaning device according to an embodiment of the present disclosure;
Figure 5 is a longitudinal cross-sectional view of the cleaning device of Figure 4, the cross-section being taken along the dotted line identified with the letter "A" in Figure 4;
Figure 6 is a front-side view of a cleaning device according to an embodiment of the present disclosure; and,
Figure 7 is a rear-side view of a cleaning device according to an embodiment of the present disclosure.

### DETAILED DESCRIPTION

The following embodiments are merely illustrative and are not intended to be limiting. It will be appreciated that various modifications and/or alterations to the embodiments described herein may be made without departing from the disclosure and any modifications and/or alterations are within the scope of the contemplated disclosure.

In an aspect, the present disclosure provides a cleaning device for use with a feeding tube connector, the cleaning device comprising: a suction connector positioned at a first end of the cleaning device, the suction connector having a first opening at a distal end of the suction connector, the suction connector being adapted for insertion into a suction tube; a cleaning member positioned at a second end of the cleaning device, the second end being opposite to the first end, the cleaning member having a second opening at a distal end of the cleaning member, the cleaning member being adapted to clean the feeding tube connector; and, a first passage extending from the first opening of the suction connector to the second opening of the cleaning member, the first passage allowing debris to be sucked from the feeding tube connector.

In another aspect, the present disclosure provides a cleaning device further comprising a barrier positioned between the suction connector and the cleaning member, the barrier being shaped to stop advancement of the suction tube.

In another aspect, the present disclosure provides a cleaning device wherein a first diameter of the barrier is wider than a second diameter of an opening of the suction tube.

In another aspect, the present disclosure provides a cleaning device further comprising at least one protrusion adapted to scrape the debris from a moat of the feeding tube connector.

In another aspect, the present disclosure provides a cleaning device further comprising a funnel extending from the barrier, the funnel having: a dish portion; a third opening within the dish portion, the third opening being adapted to be occluded by a user; a neck connecting the dish portion to the barrier; and, a second passage extending from the third opening to the first passage.

In another aspect, the present disclosure provides a cleaning device, wherein the dish portion curves inwardly toward the third opening, wherein the second passage is in fluid communication with the third opening, wherein the first passage is in fluid communication with the second passage, and wherein suction power within the first passage is increased when the third opening is occluded.

In another aspect, the present disclosure provides a cleaning device, wherein the suction connector comprises at least one convex segment, the at least one convex segment being shaped to create a sealed connection with the suction connector.

In another aspect, the present disclosure provides a cleaning device, wherein the suction connector comprises at least two convex segments, and wherein diameters of the at least two convex segments decrease as they are positioned further from the barrier.

In another aspect, the present disclosure provides a cleaning device, wherein a diameter of each of the at least one convex segment tapers towards the distal end of the suction connector.

In another aspect, the present disclosure provides a cleaning device further comprising a shield, the shield comprising: a wall portion positioned within the first passage, the wall portion preventing debris in the first passage from falling into the feeding tube connector; at least two bridge members connecting the wall portion to an inner surface of the first passage; and, at least two gaps formed between the at least two bridge members, the at least two gaps allowing debris to be sucked through the first passage.

In another aspect, the present disclosure provides a cleaning device, wherein each of the at least two gaps are positioned between the wall portion and the inner surface of the first passage to allow debris to travel along the first passage from the cleaning member to the suction connector.

In another aspect, the present disclosure provides a cleaning device, wherein the cleaning member further comprises a cylindrical portion, and wherein a distal end of the at least one protrusion extends beyond a distal end of the cylindrical portion.

In another aspect, the present disclosure provides a cleaning device, wherein the at least one protrusion is positioned on the cylindrical portion such that rotation of the cleaning member allows the at least one protrusion to scrape off the debris from the moat.

In another aspect, the present disclosure provides a cleaning device, wherein the at least one protrusion comprises at least one distal edge, and wherein the at least one distal edge is smoothed to prevent injury to a patient.

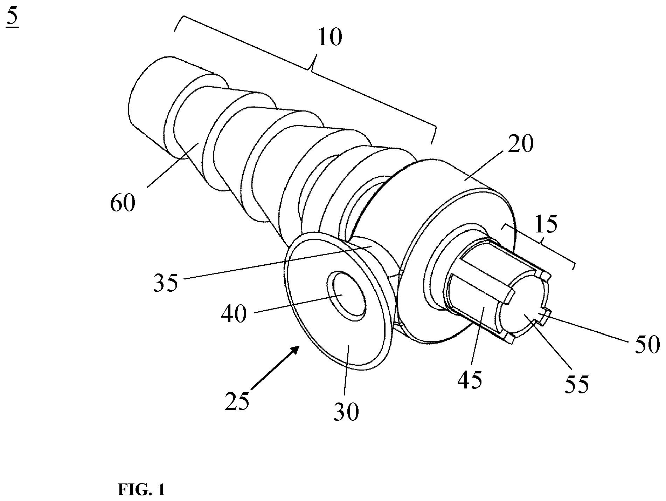

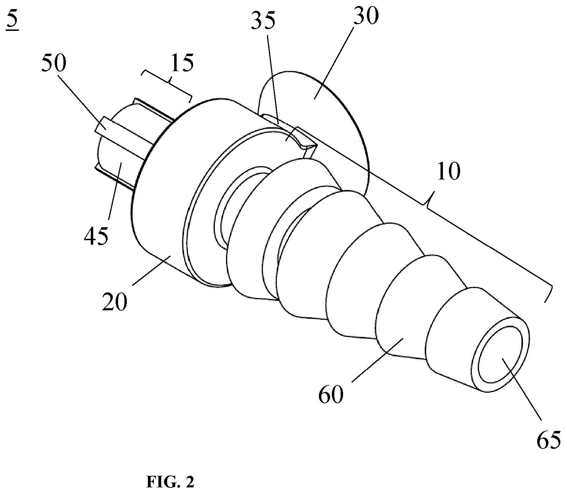

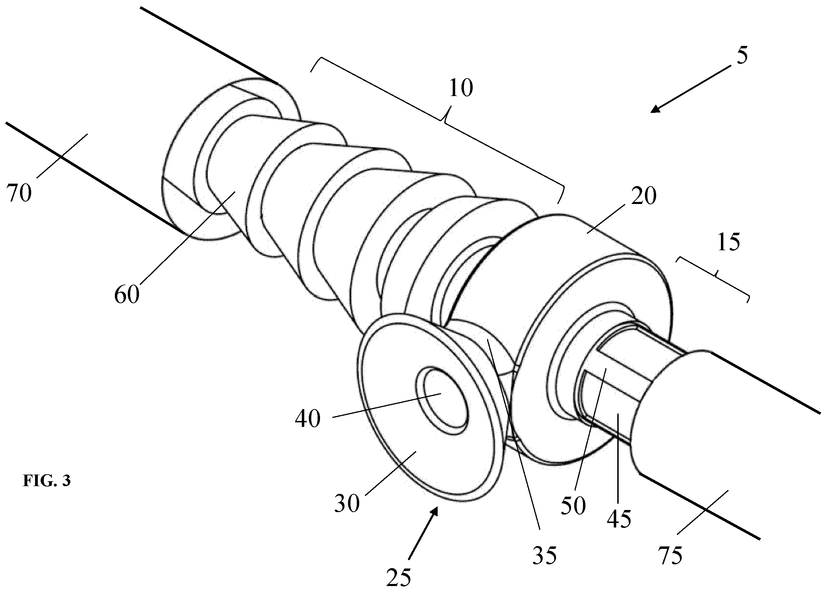

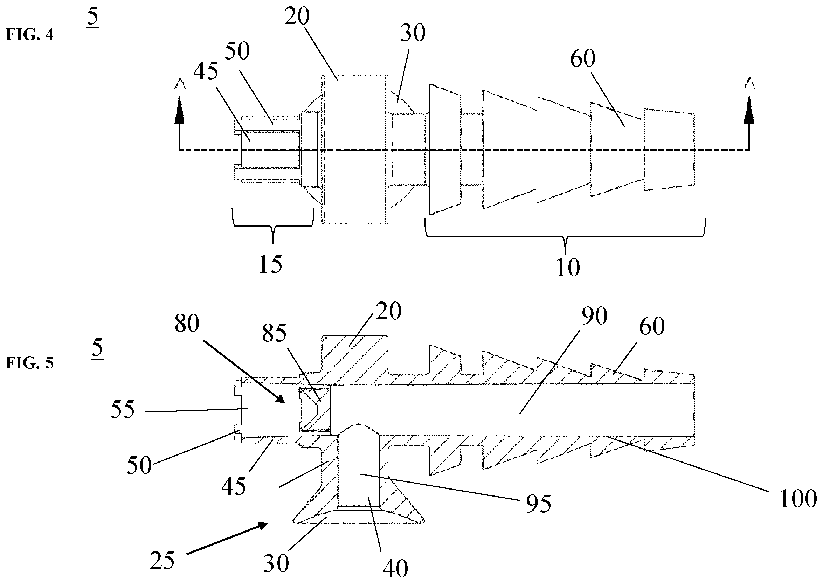

With reference to Figures 1, 2, 3, and 4 and according to an embodiment of the present disclosure, a cleaning device 5 for cleaning a feeding tube connector 75 is shown. The cleaning device 5 comprises a suction connector 10 at a first end, and a cleaning member 15 at a second end opposite to the first end. The suction connector 10 terminates at one end with a first opening 65 while the cleaning member 15 terminates at an opposite end with a second opening 55. The suction connector 10 is adapted to connect to a suction tube 70 while the cleaning member 15 is adapted to connect to the feeding tube connector 75. The suction tube 70 provides a suction source and during operation, debris from the feeding tube connector 75 is sucked through the second opening 55 and exits the cleaning device 5 through the first opening 65. The cleaning device 5 further comprises a barrier 20 that is positioned between the suction connector 10 and the cleaning member 15. The cleaning device 5 further comprises a series of convex segments 60 spanning the length of the suction connector 10 between the first opening 65 and barrier 20. As best shown in Figures 4 and 5, the convex segments 60 decrease in diameter as they are positioned further from the barrier 20. A worker skilled in the art will appreciate that this allows suction tubes 70 of varying diameters to form a tight seal around one of the convex segments 60. The present suction connector 10 can therefore accommodate a variety of suction tube 70 diameters, thereby increasing the versatility of the present cleaning device 5. Although the suction connector 10 in Figures 1, 2, 3, and 4 is illustrated with five convex segments 60, a worker skilled in the art would appreciate that any number of convex segments 60 can be arranged between the first opening 65 and barrier 20. In yet another preferred embodiment of the present disclosure, the diameter of the barrier 20 is larger than the diameter of the suction tube 70. If the diameter of the suction tube 70 is large enough to fit over all the convex segments 60 of the suction connector 10, the suction tube 70 will abut the barrier 20, thereby creating a seal between the suction tube 70 and barrier 20 to increase suction strength. Additionally, the larger diameter of the barrier 20 prevents the cleaning device 5 from entering too far into the suction tube 70.

With reference to Figures 1, 2, 3, 4, and 5, the cleaning member 15 comprises a cylindrical portion 45 and at least one protrusion 50 positioned along the cylindrical portion 45. Although the cleaning member 15 of Figure 1 is illustrated with four protrusions 50, a worker skilled in the art would appreciate that any number of protrusions 50 can be arranged on the cylindrical portion 45 of cleaning member 15. The cylindrical portion 45 and the at least one protrusion 50 are adapted to fit within a moat (not shown) of the feeding tube connector 75. The feeding tube connector 75 may be compatible with a variety of medical devices. In one embodiment of the present disclosure, the feeding tube connector 75 is a connector having an ISO Standard, such as ISO Standard 80369-3. During operation, the cleaning member 15 is inserted into the feeding tube connector 75, such that the protrusions 50 can contact the bottom of the moat (not shown) of feeding tube connector 75. The cleaning member 15 can then be rotated to scrape off any debris found at the bottom of the moat (not shown). The cleaning member 15 does not have brushes to clean the feeding tube connector 75 as brushes are prone to contamination and bristles may prohibit proper engagement between the cleaning member 15 and feeding tube connector 75. When the cleaning member 15 scrapes debris from the feeding tube connector 75, the suction connector 10 is connected to suction tube 70 such that any debris that is scraped off from the moat (not shown) of feeding tube connector 75 can be sucked through second opening 55. Furthermore, the cleaning device 5 comprises a first passage 90 that extends from the first opening 65 of the suction connector 10 to the second opening 55 of the cleaning member 15, thereby allowing scraped debris to flow from the second opening 55 to the first opening 65. A worker skilled in the art would appreciate that although the cleaning member 15 can fit into the moat (not shown) of a feeding tube connector 75, the cleaning member 15 does not occlude the opening of feeding tube connector 75, thereby decreasing the chance of contaminants being transferred from the cleaning device 5 to the feeding tube connector 75.

With reference to Figures 1, 2, 3, 4, and 5, and in a preferred embodiment of the present disclosure, the cleaning device 5 further comprises a funnel 25 that extends from barrier 20. The funnel 25 comprises a dish portion 30 that is connected to barrier 20 with a neck 35. The dish portion 30 curves inwardly towards a third opening 40 that is in fluid communication with a second passage 95 that is in turn in fluid communication with the first passage 90. During operation, a user can increase suction power by occluding the third opening 40 with, for example, their thumb. Additionally, a user may instill a liquid substance, such as a sterile saline solution or water, into the funnel 25. The liquid substance can then break up any lingering solid debris within the cleaning device 5 so that the debris can be more readily aspirated by the suction tube 70. With reference to known cleaning devices, liquid substances are applied to a male portion that is then plugged into a feeding tube connector, thereby introducing unwanted particles into the connector. A worker of skill in the art will therefore appreciate that a user may instill a liquid substance into the present cleaning device 5 without contaminating the feeding tube connector 75.

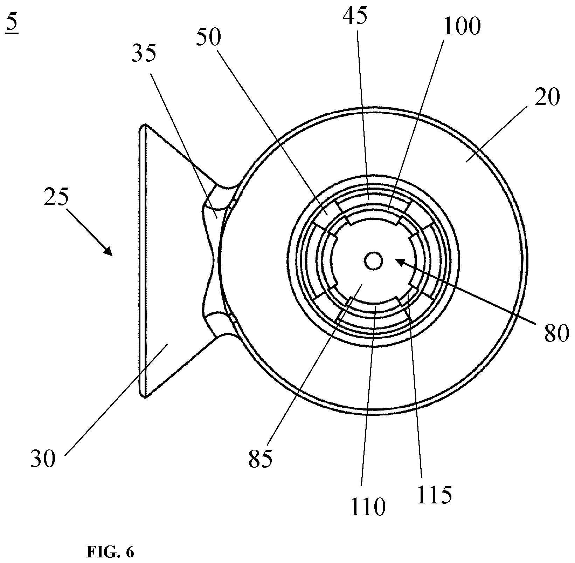

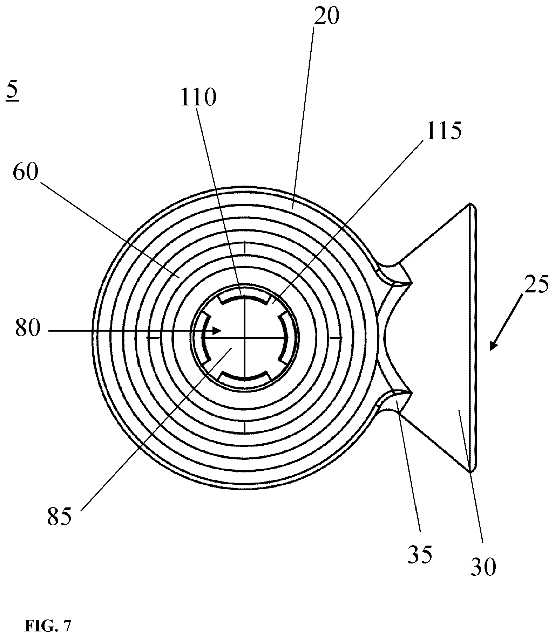

Referring to Figures 5, 6, and 7, and according to another embodiment of the present disclosure, cleaning device 5 further comprises a shield 80 comprising at least one bridge member 115 that connects a wall portion 85 of the shield 80 with an inner surface 100 of the first passage 90. The bridge member 115 creates a gap 110 between the wall portion 85 of the shield 80 and the inner surface 100 of the first passage 90 that allows debris from the second opening 55 to flow through first passage 90 and towards the first opening 65. Although the cleaning device 5 in Figures 6 and 7 are illustrated with four bridge members 115 and four gaps 110, a worker skilled in the art would appreciate that any number of bridge members 115 and gaps 110 can be arranged around the wall portion 85. The wall portion 85 of the shield 80 will block unwanted particles within the passage 90 from entering the feeding tube connector 75. A worker of skill in the art will appreciate that shield 80 is placed within the first passage 90 of the cleaning device 5 and as such, does not occlude an opening (not shown) of the feeding tube connector 75 and therefore does not introduce contaminants into the opening (not shown) of the feeding tube connector 75. In contrast, known cleaning devices have male portions that plug into feeding tube connectors, thereby introducing unwanted particles into the connectors.

Referring to the Figures 1-7, many of the features of the cleaning device 5 are illustrated with edges. A worker of skill in the art will appreciate that any one of the illustrated edges can be smoothed. Smoothed edges are advantageous to prevent accidentally scratching a patient, such as a baby. If the cleaning device 5 accidentally contacts the patient, for instance if the cleaning device 5 is accidentally dropped on the patient, smoothed edges will improve the safety of the cleaning device 5 and prevent accidental injury to the patient. In particular, any of the distal corners of the at least one protrusions 50 as shown in Figures 1-6 can be smoothed to prevent accidentally scratching the patient.

Many modifications of the embodiments described herein as well as other embodiments may be evident to a person skilled in the art having the benefit of the teachings presented in the foregoing description and associated drawings. It is understood that these modifications and additional embodiments are captured within the scope of the contemplated disclosure which is not to be limited to the specific embodiment disclosed.

## Claims

1. A cleaning device for use with a feeding tube connector, the cleaning device comprising:
a suction connector positioned at a first end of the cleaning device, the suction connector having a first opening at a distal end of the suction connector, the suction connector being adapted for insertion into a suction tube;
a cleaning member positioned at a second end of the cleaning device, the second end being opposite to the first end, the cleaning member having a second opening at a distal end of the cleaning member, the cleaning member being adapted to clean the feeding tube connector; and,
a first passage extending from the first opening of the suction connector to the second opening of the cleaning member, the first passage allowing debris to be sucked from the feeding tube connector.

2. The cleaning device of Claim 1, further comprising a barrier positioned between the suction connector and the cleaning member, the barrier being shaped to stop advancement of the suction tube.

3. The cleaning device of Claim 2, wherein a first diameter of the barrier is wider than a second diameter of an opening of the suction tube.

4. The cleaning device of any one of the preceding Claims, wherein the cleaning member comprises at least one protrusion adapted to scrape the debris from a moat of the feeding tube connector.

5. The cleaning device of any one of Claim 2 or of Claim 3 or of Claim 4 when dependent on Claim 2 or Claim 3, further comprising a funnel extending from the barrier, the funnel having:
a dish portion;
a third opening within the dish portion, the third opening being adapted to be occluded by a user;
a neck connecting the dish portion to the barrier; and,
a second passage extending from the third opening to the first passage.

6. The cleaning device of Claim 5, wherein the dish portion curves inwardly toward the third opening, wherein the second passage is in fluid communication with the third opening, wherein the first passage is in fluid communication with the second passage, and wherein suction power within the first passage is increased when the third opening is occluded.

7. The cleaning device of any one of the preceding Claims, wherein the suction connector comprises at least one convex segment, the at least one convex segment being shaped to create a sealed connection with the suction connector.

8. The cleaning device of Claim 7, wherein the suction connector comprises at least two convex segments, and wherein diameters of the at least two convex segments decrease as they are positioned further from the barrier.

9. The cleaning device of Claim 7, wherein a diameter of each of the at least one convex segment tapers towards the distal end of the suction connector.

10. The cleaning device of any one of the preceding Claims, further comprising a shield, the shield comprising:
a wall portion positioned within the first passage, the wall portion preventing debris in the first passage from falling into the feeding tube connector;
at least two bridge members connecting the wall portion to an inner surface of the first passage; and,
at least two gaps formed between the at least two bridge members, the at least two gaps allowing debris to be sucked through the first passage.

11. The cleaning device of Claim 10, wherein each of the at least two gaps are positioned between the wall portion and the inner surface of the first passage to allow debris to travel along the first passage from the cleaning member to the suction connector.

12. The cleaning device of any one of preceding claims wherein the cleaning member comprises at least one protrusion adapted to scrape the debris from a moat of the feeding tube connector, and wherein the cleaning member further comprises a cylindrical portion, and wherein a distal end of the at least one protrusion extends beyond a distal end of the cylindrical portion.

13. The cleaning device of Claim 12, wherein the at least one protrusion is positioned on the cylindrical portion such that rotation of the cleaning member allows the at least one protrusion to scrape off the debris from the moat.

14. The cleaning device of Claim 12 or 13, wherein the at least one protrusion comprises at least one distal edge, and wherein the at least one distal edge is smoothed to prevent injury to a patient.
